Europäisches Patentamt

(19) European Patent Office

Office européen des brevets

(11) **EP 0 948 558 B1**

(12) **EUROPEAN PATENT SPECIFICATION**

(45) Date of publication and mention
of the grant of the patent:
**26.11.2003 Bulletin 2003/48**

(21) Application number: **97954209.9**

(22) Date of filing: **17.12.1997**

(51) Int Cl.[7]: **C08J 5/18**, B32B 27/32,
C08J 9/28, A61L 15/22
// C08L23:08

(86) International application number:
**PCT/US97/23705**

(87) International publication number:
**WO 98/029481 (09.07.1998 Gazette 1998/27)**

(54) **STABLE AND BREATHABLE FILMS OF IMPROVED TOUGHNESS AND METHOD OF MAKING THE SAME**

STABILE ATMUNGSAKTIVE FOLIEN MIT VERBESSERTER FESTIGKEIT UND VERFAHREN ZU IHREN HERSTELLUNG

FILMS RESPIRANTS STABLES AYANT UNE TENACITE AMELIOREE, ET PROCEDE DE FABRICATION ASSOCIE

(84) Designated Contracting States:
**BE DE ES FR GB IT NL SE**

(30) Priority: **27.12.1996 US 773826**
**08.05.1997 US 853025**

(43) Date of publication of application:
**13.10.1999 Bulletin 1999/41**

(73) Proprietor: **KIMBERLY-CLARK WORLDWIDE, INC.**
**Neenah, Wisconsin 54956 (US)**

(72) Inventors:
• **HAFFNER, William, B.**
**Kennesaw, GA 30144 (US)**
• **MCCORMACK, Ann, L.**
**Cumming, GA 30131 (US)**
• **TOPOLKARAEV, Vasily, A.**
**Appleton, WI 54915 (US)**

(74) Representative:
**DIEHL GLAESER HILTL & PARTNER**
**Patentanwälte**
**Augustenstrasse 46**
**80333 München (DE)**

(56) References cited:
**EP-A- 0 659 808       EP-A- 0 779 325**
**WO-A-96/19346       US-A- 4 705 812**
**US-A- 5 522 810**

**Description**

**FIELD OF INVENTION**

The present invention is directed to breathable thermoplastic films utilizing copolymers of ethylene and at least one $C_4$-$C_8$ $\alpha$-olefin. In addition, the present invention is directed to a method of making such films.

**BACKGROUND OF THE INVENTION**

The present invention is directed to breathable thermoplastic films. Such materials have a wide variety of uses, especially in the areas of limited use and disposable items.

Films have been traditionally used to provide barrier properties in limited use or disposable items. By limited use or disposable, it is meant that the product and/or component is used only a small number of times or possibly only once before being discarded. Examples of such products include, but are not limited to, surgical and health care related products such as surgical drapes and gowns, disposable work wear such as coveralls and lab coats and personal care absorbent products such as diapers, training pants, incontinence garments, sanitary napkins, bandages, wipes and the like. In personal care absorbent products such as infant diapers and adult incontinence products, films are used as the outer covers with the purpose of preventing body wastes from contaminating the clothing, bedding and other aspects of the surrounding environment of use. In the area of protective apparel including hospital gowns, films are used to prevent cross exchange of microorganisms between the wearer and the patient.

US Patent 4,705,812 discloses a process for producing porous films which comprises melting a resin composition consisting essentially of (1) 100 parts by weight of a high-pressure-processed low-density polyethylene resin having a melt-index of 0.5 to 7 and a density of 0.915 to 0.935, a linear low-density polyethylene resin having a melt-index of 0.5 to 8.5 and a density of 0.915 to 0.935, or a mixture thereof and (2) 50 to 500 parts by weight of barium sulface having an average particle diameter of 0.1 to 7 $\mu$m; forming the molten resin composition into a film; and then stretching the film at least uniaxially by a factor of 1.5 to 7.

While these films can be effective barriers, they are not aesthetically pleasing because their surfaces are smooth and either feel slick or tacky. They are also visually flat and "plasticy" thereby making them less desirable in apparel applications and other uses where they are in contact with human skin. It would be more preferable if these items were more cloth-like from both a tactile and visual standpoint. For example, infant diapers that have the feel and appearance of traditional cloth undergarments are perceived as premium products and may, in some cases, overcome the tendency to believe that they need to be covered by outer garments for aesthetic reasons. Garment-like adult incontinence products could improve the self-image of the incontinent individual. In addition, more garment-like isolation gowns would help the hospital environment feel less foreign and threatening to the patient and increase the comfort of the wearer. It is also preferable to have films that can make an outercover material with more elastic give and recovery to provide better fit and comfort.

Lamination of films has been used to create materials which are both liquid-impervious and somewhat cloth-like in appearance and texture. The outer covers on disposable diapers are but one example. In this regard, reference may be had to coassigned U.S. Patent No. 4,818,600 dated April 4, 1989 and U.S. Patent No. 4,725,473 dated February 16, 1988. Surgical gowns and drapes are other examples. See, in this regard, coassigned U.S. Patent No. 4,379,102 dated April 5, 1983.

A primary purpose of the film in such laminations is to provide barrier properties. There is also a need for such laminates to be breathable so that they have the ability to transmit moisture vapor. Apparel made from laminates of these breathable or microporous films are more comfortable to wear by reducing the moisture vapor concentration and the consequent skin hydration underneath the apparel item. However, the pore size in breathable films cannot be too large, especially in protective apparel applications where chemical vapor penetration presents a contamination risk to the wearer.

The conventional process for obtaining a breathable microporous film has been to stretch a thermoplastic film containing filler. Microvoids are created by the filler particles during the stretching process. The film is usually heated prior to these drawing processes to make the film more plastic or malleable. This drawing or stretching also orients the molecular structure within the film which increases its strength and durability. The molecular orientation caused by stretching is desired to improve durability.

A film can be stretched in the machine-direction or the cross-machine direction. Stretching the film in the cross direction is particularly challenging because forces must be applied to the edges of the film to cause it to elongate width-wise. Tenter frames are commonly used. In contrast, stretching the film in the machine direction is relatively easy. It is only necessary to increase the draw, or speed ratio, between two rollers while the film is in the heated or plastic state. There is a durability problem, however, with uni-directionally-stretched films, be it machine direction or cross-direction. Unidirectional stretching causes molecular orientation only in the stretched direction. This results in films that are easily torn or split along that dimension. For example, a machine-directionally oriented film has a propensity to split or tear along the machine direction. Also, the tensile characteristics of the (machine-directionally stretched) film are dramatically increased in the machine direction, but the tensile strength in the cross-direction is significantly inferior to that of the machine direction. Thus, for example, if at the same time that the CD strength of the film decreases, the CD break elongation is also reduced, the film can split very easily in use, and an article made with it, such as a diaper, may leak, obviously an undesirable effect.

These durability problems with uni-directionally stretched or oriented films are well known. Two approaches are commonly used to obviate the product durability problems resulting from these highly isotropic strength characteristics. The first is to stretch-orient the film in both the machine and cross direction. Films that have been biaxially stretched have more balanced strength properties. The second approach is to combine into a laminate one layer of machine directionally oriented film with one layer of cross-directionally oriented film.

One other manufacturing issue is the strength of "aged" films. In commercial manufacturing operations, "fresh" films such as newly extruded films are generally not available for orientation. Extruded films are often set aside or stored for later orientation, usually at room temperature. During this storage period, a change in morphology of the polymer may occur, which change could be the cause of film property changes. Orientation of such aged film often results in products with lower durability characteristics such as lower CD Peak Strain (or cross directional break elongation), a critical property, for example, for the durability of a diaper outer cover made from this film.

There is therefore a need for an elastic breathable film and process that provides a film with both the cloth-like aesthetics and the durability and comfort that are desired.

## SUMMARY OF THE INVENTION

The present invention relates to a breathable thermoplastic film that includes a linear low density polyethylene resin material including copolymers of ethylene and $C_4$-$C_8$" olefin monomer and at least one filler comprising from 40 to 65 % by weight of said filled resin material of calcium carbonate and wherein said calcium carbonate includes a plurality of particles having a fatty acid coating, wherein the filler has a particle size that contributes to pore formation. The film has a water vapor transmission rate of from 300-4500 $g/m^2$-24 hr and a critical break elongation 90° to the direction of orientation of greater than 150%. In one application, where the minimum desired water vapor transmission rate is about 1,500 $g/m^2$/24 hours, the amount of filler present is about 48 weight percent.

The present invention also is directed to a process for preparing the breathable film of the present invention, including providing a polymeric resin including a linear low density polyethylene resin material; adding to the resin from 40 to 65 % by weight of said filled resin material of calcium carbonate and wherein said calcium carbonate includes a plurality of particles having a fatty acid coating, having a particle size that contributes to pore formation to form a filled resin; forming a film having a first length from the filled resin; and stretching the film to form a microporous film. The process of the invention is applicable to films formed by various processes, i.e., cast or blown films. In one embodiment of the invention, the microporous film is stretched to a second length that is from about 160 to about 400% of the first length. In another embodiment, the film is annealed following stretching.

The film of the present invention has a water vapor transmission rate of from 300 to 4,500 grams per square meter per 24 hours (measured by ASTM Standard Test E 96-80 with Celgard® 2500 as control).

Such films have a wide variety of uses including, but not limited to, applications in personal care absorbent articles including diapers, training pants, sanitary napkins, incontinence devices, bandages. These same films also may be used in items such as surgical drapes and gowns as well as various articles of clothing either as the entire article or simply as a component thereof.

## BRIEF DESCRIPTION OF THE DRAWINGS

Figure 1 is a schematic side view of a process for forming a film according to the present invention.

Figure 2 is a cross-section side view of a film/nonwoven laminate according to the present invention.

Figure 3 is a partially cut away top plan view of an exemplary personal care absorbent article, in this case a diaper, which may utilize a film made according to the present invention.

## DETAILED DESCRIPTION OF THE PREFERRED EMBODIMENT

The present invention is directed to breathable thermoplastic films that include copolymers of ethylene and $C_4$-$C_8$ $\alpha$-olefin monomer.

One particularly useful example is known as "super-octene." The term "super-octene" as used herein includes those linear low density polyethylene (LLDPE) materials that are produced by the polymerization of ethylene and 1-octene comonomer and designated Dowlex® NG brand ("NG resin"), available from Dow Chemical Corporation of Midland, Michigan. The "super-octene" resin are made with an improved catalyst system other than "metallocene" or Insite®. Suitable "super-octene" resins useful in the present invention include, for example, Dowlex® NG 3347A and Dowlex® NG 3310, both of which contain about 7% octene (nominal weight %), 93% ethylene. While not wishing to be bound by the following theory, it is postulated that the improved catalyst regulates the molecular weight/molecular weight distribution as well as comonomer placement and branching on the polymer molecule more precisely than conventional catalysts. It is possible, for example, that as a result of the improved technology, the NG resins have narrower molecular weight distribution, more homogeneous branching distribution as well as smaller highly branched low density and unbranched high density fractions. The physical characteristics of unfilled films made from super-octene resins do not distinguish this resin from conventional LLDPE resins, as illustrated in Table A below. Table A lists physical data of Dowlex® NG 3347A and, for comparison, data of certain "conventional" LLDPE resins, Dowlex® 2045 and 2244A.

TABLE A

|  |  | NG 3347A | Dowlex® 2045 | Dowlex® 2244A |
|---|---|---|---|---|
| Melt index, gm/10 min. (an indication of MW) | | 2.3 | 1.0 | 3.3 |
| Density, gm/cc | | 0.917 | 0.920 | 0.9155 |
| Film extrusion method | | blown | blown | cast |
| (a) | Tensile yield, MPa, MD/CD | 9.0/8.3 | 10.3/11.0 | 7.6/6.9 |
| (b) | Break tensile, MPa, MD/CD | 60/34 | 57.9/40.0 | 54.5/37.3 |
| (c) | Break elongation, %, MD/ CD | 550/750 | 600/750 | 580/780 |
| (d) | Elmendorf tear, gm, MD/CD | 330/540 | 375/750 | 350/580 |

As can be seen from Table A above, the typical properties ((a) through (d)) of unfilled films from these various resins are not remarkably different. Minor variations could be explained by variations in melt index and $\alpha$-olefins density or crystallinity.

Other ethylene-based copolymers with $C_4$-$C_8$ $\alpha$-olefins are also useful in the present invention including, for example, materials commercially from Exxon Corporation under the brand name Exact™ These materials are all prepared with a "new" or "improved" catalyst system with respect to the metallocene or similar single-site catalysts.

Other suitable ethylene-based copolymers with $C_4$-$C_8$ $\alpha$-olefin monomers of the present invention include nonelastic metallocene-catalyzed polymers. The term "metallocene-catalyzed polymers" as used herein includes those polymer materials that are produced by the polymerization of at least ethylene using metallocenes or constrained geometry catalysts, a class of organometallic complexes, as catalysts. For example, a common metallocene is ferrocene, a complex with a metal sandwiched between two cyclopentadienyl (Cp) ligands. Metallocene process catalyses include bis(n-butylcyclopentadienyl)titanium dichloride, bis(n-butylcyclopentadienyl)zirconium dichloride, bis(cyclopentadienyl)scandium chloride, bis(indenyl)zirconium dichloride, bis(methylcyclopentadienyl)titanium dichloride, bis(methylcyclopentadienyl)zirconium dichloride, cobaltocene, cyclopentadienyltitanium trichloride, ferrocene, hafnocene dichloride, isopropyl(cyclopentadienyl,-1-flourenyl)zirconium dichloride, molybdocene dichloride, nickelocene, niobocene dichloride, ruthenocene, titanocene dichloride, zirconocene chloride hydride, zirconocene dichloride, among others. A more exhaustive list of such compounds is included in U.S. Patent 5,374,696 to Rosen et al. and assigned to the Dow Chemical Company. Such compounds are also discussed in U.S. Patent 5,064,802 to Stevens et al. and also assigned to Dow.

The metallocene process, and particularly the catalysts and catalyst support systems are the subject of a number of patents. U.S. Patent 4,542,199 to Kaminsky et al. describes a procedure wherein MAO is added to toluene, the metallocene catalyst of the general formula (cyclopentadienyl)2MeRHal wherein Me is a transition metal, Hal is a halogen and R is cyclopentadienyl or a C1 to C6 alkyl radical or a halogen, is added, and ethylene is then added to form polyethylene. U.S. Patent 5,189,192 to LaPointe et al. and assigned to Dow Chemical describes a process for preparing addition polymerization catalysts via metal center oxidation. U.S. Patent 5,352,749 to Exxon Chemical Patents, Inc. describes a method for polymerizing monomers in fluidized beds. U.S. Patent 5,349,100 describes chiral metallocene compounds and preparation thereof by creation of a chiral center by enantioselective hydride transfer. Co-catalysts are materials such as methylaluminoxane (MAO) which is the most common, other alkylaluminums and boron containing compounds like tris-(pentafluorophenyl)boron, lithium tetrakis(pentafluorophenyl)boron, and dimethylanilinium tetrakis(pentafluorophenyl)boron. Research is continuing on other co-catalyst systems or the possibility of minimizing or even eliminating the alkylaluminums because of handling and product contamination issues. The important point is that the metallocene catalyst be activated or ionized to a cationic form for reaction with the monomer(s) to be polymerized.

The metallocene-catalyzed ethylene-based polymers used in the present invention impart stretch and recovery properties to the film. Preferably, the metallocene catalyzed ethylene-based polymer is selected from copolymers of ethylene and 1-butene, copolymers of ethylene and 1-hexene, copolymers of ethylene and 1-octene and combinations thereof. In particular, preferred materials include Affinity™ brand metallocene-derived copolymers of ethylene and 1-octene, both available from Dow Plastics of Freeport, Texas. Also preferred are Exact™ brand metallocene-derived copolymers of ethylene and 1-butene and copolymers of ethylene and 1-hexene, available from Exxon Chemical Company of Houston, Texas. In general, the metallocene-derived ethylene-based polymers of the present invention have a density of at least 0.900 g/cc.

At least one copolymer of ethylene and $C_4$-$C_8$ $\alpha$-olefin monomer is the major polymeric component of the film of the present invention. Preferably, the film of the present invention contains at least 30 percent, more preferably about 40-50 percent by weight of the filled film composition. Other polymeric components may also be present so long as they do not adversely affect the desired characteristics of the film.

In addition to the polymeric material, the film layer also includes a filler which enables development of micropores during orientation of the film. As used herein a "filler" is meant to include particulates and other forms of materials which can be added to the polymer and which will not chemically interfere with or adversely affect the extruded film but is able to be uniformly dispersed throughout the film. Generally, the fillers will be in particulate form and usually will have somewhat of a spherical shape with average particle sizes in the range of about 0.5 to about 8 microns. The film contains from 40 to 65 percent calcium carbonate filler including a plurality of particles having a fatty acid coating, based upon the total weight of the film layer. More preferably, from about 45 to about 55 percent of filler is present in the film. Both organic and inorganic fillers are contemplated to be within the scope of the present invention provided that they do not interfere with the film formation process, the breathability of the resultant film or its ability to bond to another layer such as a fibrous polyolefin nonwoven web.

Examples of fillers which may be used in addition to calcium carbonate ($CaCO_3$), include various kinds of clay, silica ($SiO_2$), alumina, barium sulfate, sodium carbonate, talc, magnesium sulfate, titanium dioxide, zeolites, aluminum sulfate, cellulose-type powders, diatomaceous earth, magnesium sulfate, magnesium carbonate, barium carbonate, kaolin, mica, carbon, calcium oxide, magnesium oxide, aluminum hydroxide, pulp powder, wood powder, cellulose derivative, polymer particles, chitin and chitin derivatives. The additional filler particles may optionally be coated with a fatty acid, such as stearic acid or behenic acid which may facilitate the free flow of the particles (in bulk) and their ease of dispersion into the polymer matrix.

Generally, it has been possible to produce films with a water vapor transmission rate (WVTR) of at least about 300 grams per square meter per 24 hours, measured by the ASTM E-96-80 WVTR test (using Celgard® 2500 as control). In general, factors that affect the amount of breathability include the amount of filler, the film stretching conditions (e.g., whether it is performed at ambient or elevated temperatures), orientation ratio, and film thickness. The WVTR of the film of the present invention that may be used as a component in a limited-use or disposable item is from 300 to 4,500, and, in one application, preferably at least about 1,500 $g/m^2/24$ hrs. In addition, the preferred films of the present invention, when stretched in the machine direction, have superior extensibility and increased resistance to failure around film flaws.

These properties can be obtained by first preparing a polymeric resin of a super-octene LLDPE resin, filling the resin with filler, extruding a film from the filled resin and thereafter stretching or orienting the filled film in at least one direction, usually, the machine direction. As explained in greater detail below, the resultant film is microporous and has increased strength properties in the orientation direction.

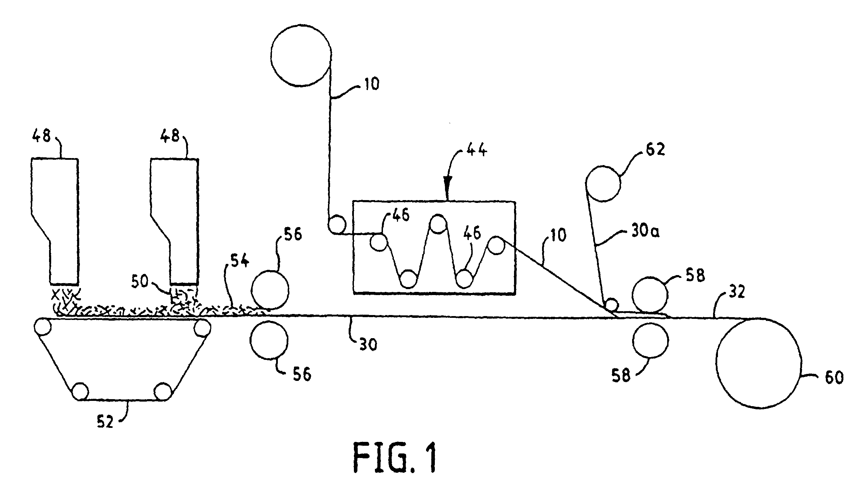

Processes for forming filled films and orienting them are well-known to those skilled in the art. In general, a process for forming oriented filled film 10 is shown in Figure 1 of the drawings. Film 10 is unwound and directed to a film stretching unit 44 such as a machine direction orienter, which is a commercially available device from vendors such as the Marshall and Williams Company of Providence, Rhode Island. Such an apparatus 44 has a plurality of stretching rollers 46 moving at progressively faster speeds relative to the pair disposed before them. These rollers 46 apply an amount of stress and thereby progressively stretched filled film 10 to a stretch length in the machine direction of the film which is the direction of travel of filled film 10 through the process as shown in Figure 1. The stretch rollers 46 may be heated for better processing. Preferably, unit 44 also includes rollers (not shown) upstream and/or downstream from the stretch rollers 46 that can be used to preheat the film 10 before orienting and/or anneal (or cool) it after stretching. The purpose of the annealing is to stabilize the film so that it will shrink less or not at all when it is exposed to elevated temperatures during subsequent processing, storage, transportation, or product use.

Uniaxial orientation is a well known art in the plastics film industry. Films are often oriented to enhance their strength and other physical properties. The most common and simplest kind of uniaxial orientation is in the machine direction (MD) on equipment often called machine direction orientor, or MDO for short. Various MDO designs are used in the industry, all of which use temperature-controlled rollers for heating or cooling and transport of the film being processed. Stretching is accomplished between the slow nip and the fast nip, the slow nip holding the film back, and the fast nip accelerating the film causing it to become longer and at the same time thinner and somewhat narrower (necked). In practical terms the degree of orientation is usually described as the stretch ratio, such as 3X or 4X, which is the ratio of the surface speed of fast nip to the surface speed of the slow nip. The slow nip is generally preceded by preheat rolls which can heat the film to the desired stretching temperature, and the fast nip is followed by rolls to heat the film to some annealing temperature. Cooling roll(s) may be used to cool the stretched film before further processing.

Polymer films are oriented above the glass transition temperature and below the melting temperature of the polymers used. Good film properties can be obtained at relatively low stretch temperatures, such as room temperature. However, higher temperature may be used for ease of processing and to allow practical processing speeds. For example, the preheat and slow nip may be at 71,1°C (160°F), fast nip and first annealing roll may be varied from room temperature to about 101,7°C (215°F), and the last annealing roll may be at about 93,3, 98,9, or 101,7°C (200, 210, or 215°F). Processing speeds may be at about 24,24-30,3m (80-100 feet) per minute (fpm) on the slow nip, and about 96,96-128,8 mpm (320-425 (fpm)) on the fast nip.

The useful degree of stretching for breathable films is somewhat different for different polymers used. To avoid unstretched segments or spots in the film, that is, to make fully oriented ("whitened") film, the lowest stretch ratio is preferably about 3X. Good results may be obtained at about 4X-4.25X. If a film is "overstretched" (generally above about 5X), it could become excessively splitty. At the stretched length, a plurality of micropores form in the film 10. If desired, film 10 is directed out of apparatus 44 so that the stress is removed to allow the stretched film 10 to relax.

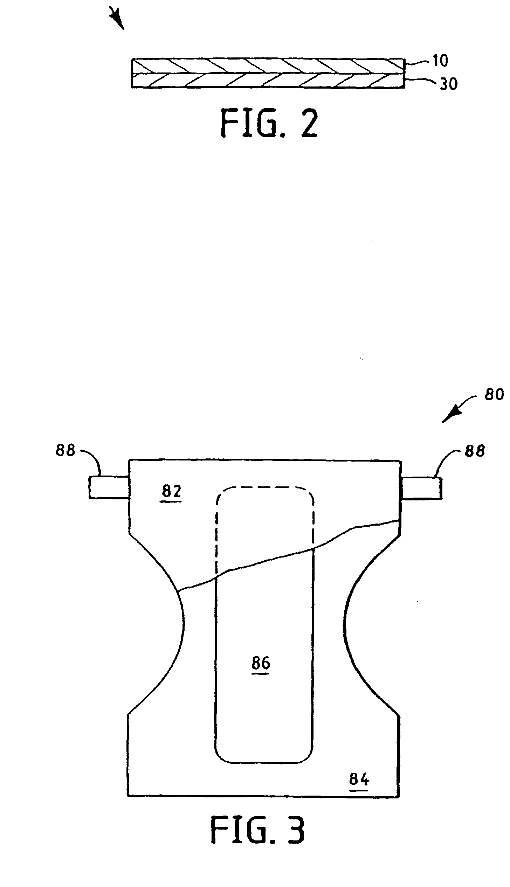

Oftentimes it may be desirable to laminate filled film 10 to one or more substrates or support layers 20 such as is shown in Figure 2. Lamination of film may enhance the strength and thus durability of the film. If desired, filled film 10 may be attached to one or more support layers 30 to form a laminate 32. Referring again to Figure 1, a conventional fibrous nonwoven web-forming apparatus 48, such as a pair of spunbond machines, is used to form the support layer 30. The long, essentially continuous fibers 50 are deposited onto a forming wire 52 as an unbonded web 54 and the unbonded web 54 is then sent through a pair of bonding rolls 56 to bond the fibers together and increase the tear strength of the resultant web support layer 30. One or both of the rolls are often heated to aid in bonding. Typically, one of the rolls 56 is also patterned so as to impart a discrete bond pattern with a prescribed bond surface area to the web 30. The other roll is usually a smooth anvil roll but this roll also may be patterned if so desired. Once filled film 10 has been sufficiently stretched and the support layer 30 has been formed, the two layers are brought together and laminated to one another using a pair of laminating rolls or other means 58. As with the bonding rolls 56, the laminating rolls 58 may be heated. Also, at least one of the rolls may be patterned to create a discrete bond pattern with a prescribed bond surface area for the resultant laminate 32. Generally, the maximum bond point surface area for a given area of surface on one side of the laminate 32 will not exceed about 50 percent of the total surface area . There are a number of discrete bond patterns which may be used. See, for example, Brock et al., U.S. Patent No. 4,041,203. Once the laminate 32 exists the laminating rolls 58, it may be wound up into a roll 60 for subsequent processing. Alternatively, the laminate 32 may continue in-line for further processing or conversion.

While the support layers 30 and film 10 shown in Figure 1 were bonded together through thermal point bonding, other bonding means can also be used. Suitable alternatives include, for example, adhesive bonding and the use of tackifiers. In adhesive bonding, an adhesive such as a hot melt adhesive is applied between the film and fiber to bind the film and fiber together. The adhesive can be applied by, for example, melt spraying, printing or meltblowing. Various types of adhesives are available, including those produced from amorphous polyalphaolefins, ethylene vinyl acetate-based hot melts, and Kraton® brand adhesives available from Shell Chemical of Houston, Texas and Rextac™ Brand Adhesives from Rexene of Odessa, Texas.

When the film and support layer(s) is bonded with tackifiers, the tackifier may be incorporated into the film itself. The tackifier essentially serves to increase adhesion between the film and fiber layers. The film and fiber laminate may subsequently be thermally point-bonded, although generally very little heat is required since the tackifier tends to increase the pressure sensitivity of the film and a bond somewhat like and adhesive bond can be formed. Examples of useful tackifiers include Wingtack™ 95, available from Goodyear Tire & Rubber Co. of Akron, Ohio, and Escorez™ 5300, available from Exxon Chemical Company of Houston, Texas.

The support layers 30 as shown in Figure 2 are fibrous nonwoven webs. The manufacture of such fibrous nonwoven webs is known. Such fibrous nonwoven webs can add additional properties to filled film 10, such as a more soft, cloth-like feel. This is particularly advantageous when filled film 10 is being used as a barrier layer to liquids in such applications as outer covers for personal care absorbent articles and as barrier materials for hospital, surgical, and clean room applications such as, for example, surgical drapes, gowns and other forms of apparel. Attachment of the support layers 30 to the filled film 10 may be by the use of a separate adhesive such as hot-melt and solvent based adhesives or through the use of heat and/or pressure (also known as thermal bonding) as with heated bonding rolls.

The support layer in a laminate containing the film layer of the present invention can be necked polypropylene spunbond, crimped polypropylene spunbond, bonded carded webs, elastomeric spunbond or meltblown fabrics produced from elastomeric resins. A particularly advantageous support layer is a fibrous nonwoven web. Such webs may be formed from a number of processes including, but not limited to, spunbonding, meltblowing and bonded carded web processes. Meltblown fibers are formed by extruding molten thermoplastic material through a plurality of fine, usually circular, capillaries as molten threads or filaments into a high velocity usually heated gas stream such as air, which attenuates the filaments of molten thermoplastic material to reduce their diameters. Thereafter, the meltblown fibers are carried by the high velocity usually heated gas stream and are deposited on a collecting surface to form a web of randomly dispersed meltblown fibers. The meltblown process is well-known and is described in various patents and publications, including NRL Report 4364, "Manufacture of Super-Fine Organic Fibers" by B. A. Wendt, E. L. Boone and D. D. Fluharty; NRL Report 5265, "An Improved Device For The Formation of Super-Fine Thermoplastic Fibers" by K. D. Lawrence, R. T. Lukas, J. A. Young; U.S. Patent No. 3,676,242, issued July 11, 1972, to Prentice; and U.S. Patent No. 3,849,241, issued November 19, 1974, to Buntin, et al.

Spunbond fibers are formed by extruding a molten thermoplastic material as filaments from a plurality of fine, usually circular, capillaries in a spinnerette with the diameter of the extruded filaments then being rapidly reduced, for example, by non-educative or educative fluid-drawing or other well-known spunbonding mechanisms. The production of spunbond nonwoven webs is illustrated in patents such as Appel et al., U.S. Patent No. 4,340,563; Matsuki, et al., U.S. Patent No . 3,802,817; Dorschner et al., U.S. Patent No. 3,692,618; Kinney, U.S. Patent Nos . 3,338,992 and 3,341,394; Levy, U.S. Patent No. 3,276,949; Peterson, U.S. Patent No. 3,502,538; Hartman, U.S. Patent No. 3,502,763; Dobo et al., U.S. Patent No. 3,542,615; and Harmon, Canadian Patent No. 803,714.

A plurality of support layers 30 also may be used. Examples of such materials can include, for example, spunbond/meltblown laminates and spunbond/meltblown/spunbond laminates such as are taught in Brock et al., U.S. Patent No. 4,041,203.

Bonded carded webs are made from staple fibers which are usually purchased in bales. The bales are placed in a picker which separates the fibers. Next the fibers are sent through a combing or carding unit which further breaks apart and aligns the staple fibers in the machine direction so as to form a machine direction-oriented fibrous nonwoven web. Once the web has been formed, it is then bonded by one or more of several bonding methods. One bonding method is powder bonding wherein a powdered adhesive is distributed throughout the web and then activated, usually by heating the web and adhesive with hot air. Another bonding method is pattern bonding wherein heated calender rolls or ultrasonic bonding equipment is used to bond the fibers together, usually in a localized bond pattern though the web can be bonded across its entire surface if so desired. When using bicomponent staple fibers, through-air bonding equipment is, for many applications, especially advantageous.

The process shown in Figure 1 also may be used to create a three layer laminate. The only modification to the previously described process is to feed a supply 62 of a second fibrous nonwoven web support layer 30a into the laminating rolls 58 on a side of filled film 10 opposite that of the other fibrous nonwoven web support layer 30. As shown in Figure 1, one or both of the support layers may be formed directly in-line, as is support layer 30. Alternatively, the supply of one or both support layers may be in the form of a pre-formed roll 62, as is support layer 30a. In either event, the second support layer 30a is fed into the laminating rolls 58 and is laminated to filled film 10 in the same fashion as the first support layer 30.

As has been stated previously, filled film 10 and the breathable laminate 32 may be used in a wide variety of applications not the least of which includes personal care absorbent articles such as diapers, training pants, incontinence devices and feminine hygiene products such as sanitary napkins. An exemplary article 80, in this case a diaper, is shown in Figure 3 of the drawings. Referring to Figure 3, most such personal care absorbent articles 80 include a liquid permeable top sheet or liner 82, a back sheet or outercover 84 and an absorbent core 86 disposed between and contained by the top sheet 82 and back sheet 84. Articles 80 such as diapers may also include some type of fastening means 88 such as adhesive fastening tapes or mechanical hook and loop type fasteners to maintain the garment in place on the wearer. The fastening system may contain stretch material to form "stretch ears" for greater comfort.

Filled film 10 by itself or in other forms such as the film/support layer laminate 32 may be used to form various portions of the article including, but not limited to, stretched ears, the top and the back sheet 84. If the film or laminate is to be used as the liner 82, it will have to be apertured or otherwise made to be liquid permeable. When using a film/nonwoven laminate as the outercover 84, it is usually advantageous to place the nonwoven side facing out away from the user. In addition, in such embodiments it may be possible to utilize the nonwoven portion of the laminate as the loop portion of the hook and loop combination.

Other uses for the filled film and breathable film/support layer laminates according to the present invention include, but are not limited to, medical protective articles such as surgical drapes and gowns, as well as wipers, barrier materials and articles of clothing or portions thereof including such items as workwear and lab coats.

The advantages and other characteristics of the present invention are best illustrated by the following examples:

## EXAMPLES

Eight resin compositions listed in Table I below were compounded. Films were formed and stretched in a machine-direction orientator in accordance to the condition listed in Table I below.

TABLE I

| FILM | COMPOSITION | FILM TYPE | STRETCH CONDITION | | | FINAL BASIS |
|------|-------------|-----------|---------|---------|---------|-------------|
| | | | STRETCH RATIO | TEMPERATURE °F (°C) | | WEIGHT |
| | | | | STRETCH | ANNEAL | (g/m$^2$) |
| A | 55% Supercoat™ $CaCO_3$ (1 micron average particle size) from English China Clay America Inc. Sylacauga, Alabama<br><br>40% Dowlex®NG3347A (2.3MI) octene LLDPE Dow Chemical Corp. Midland, MI<br><br>5% Exxon LD-134.09 (2MI) LOPE Exxon Chemical Co. Houston, TX | Blown | 4.25 | 160 (71.11°C) | 215 (101.67°C) | 15 |
| B | 55% Supercoat™ $CaCO_3$ (1 micron average particle size) from English China Clay America Inc. Sylacauga, Alabama<br><br>30% LLDPE blend: Dowlex® 2517 and Dowlex® 2532(1:4) 10MI (melt index)<br><br>15% Dowlex® LLDPE 2045 1MI (melt index) | Blown | 4.25 | 160 (71.11°C) | 215 (101.67°C) | 12 |

TABLE I (continued)

| FILM | COMPOSITION | FILM TYPE | STRETCH CONDITION | | | FINAL BASIS |
|---|---|---|---|---|---|---|
| | | | STRETCH RATIO | TEMPERATURE °F (°C) | | WEIGHT |
| | | | | STRETCH | ANNEAL | (g/m$^2$) |
| C | 48% Supercoat™ CaCO$_3$ (1 micron average particle size) from English China Clay America Inc. Sylacauga, Alabama<br><br>47% Dowlex®NG3347A octene LLDPE (2.3MI)<br><br>5% Dow 640 LDPE (2MI) | Blown | 4.0 | 160 (71.11°C) | 220 (104.44°C) | 18 |
| D | 48% Supercoat™ CaCO$_3$ (1 micron average particle size) from English China Clay America Inc. Sylacauga, Alabama<br><br>47% Dowlex®NG3310 octene LLDPE (3.5MI)<br><br>5% Dow 722 LDPE (8MI) | Cast | 4.0 | 160 (71.11°C) | 215 (101.67°C) | 16 |
| E | 47% Supercoat™ CaCO$_3$ CaCO$_3$ (1 micron average particle size) from English China Clay America Inc Sylacauga, Alabama<br><br>48% Dow Affinity™ PL-1845 octene LLDPE (3.5MI) Metallocene $\rho$ = 0.910g/cc<br><br>5% Dow 5004 LDPE (4MI) | Cast | 3.25 | 160 (71.11°C) | 190 (87.79°C) | 8 |

TABLE I   (continued)

| FILM | COMPOSITION | FILM TYPE | STRETCH CONDITION | | | FINAL BASIS |
|------|-------------|-----------|--------|--------|--------|-------------|
| | | | STRETCH RATIO | TEMPERATURE °F (°C) | | WEIGHT |
| | | | | STRETCH | ANNEAL | (g/m$^2$) |
| F | 48% Supercoat™ CaCO$_3$ CaCO$_3$ (1 micron average particle size) from English China Clay America Inc. Sylacauga, Alabama<br><br>52% Dow Affinity™ PL 1280 octene LLDPE (5MI) $\rho$=0.900g/cc | Cast | 4.25 | 160 (71.11°C) | 180 (82.22°C) | 15 |
| G | 55% Supercoat™ CaCO$_3$ CaCO$_3$ (1 micron average particle size) from English China Clay America Inc. Sylacauga, Alabama<br><br>15% Himont X-11395-5-1 Catalloy™ Reactor TPO-5MFR (melt flow rate) Himont Incorporated, Wilmington, DE<br><br>15% LLDPE blend: Dowlex® 2517 and Dowlex® 2532 (1: 4) 10MI (melt index)<br><br>15% Dowlex® 2045 1MI (melt index) | Blown | 4.25 | 160 (71.11°C) | 215 (101.67°C) | 17 |

TABLE I   (continued)

| FILM | COMPOSITION | FILM TYPE | STRETCH CONDITION | | | FINAL BASIS |
| --- | --- | --- | --- | --- | --- | --- |
| | | | STRETCH RATIO | TEMPERATURE °F (°C) | | WEIGHT |
| | | | | STRETCH | ANNEAL | (g/m$^2$) |
| H | 55% Supercoat™ CaCO$_3$ CaCO$_3$ (1 micron average particle size) from English China Clay America Inc. Sylacauga, Alabama | Blown | 4.25 | 160 | 215 | 14 |
| | 45% Union Carbide 6D81 (copolymer of propylene and 5.5% ethylene) (5.5 MFR) Union Carbide Corp. Danbury, CT | | | | | |

WVTR and cross-machine direction break elongation characteristics of each stretched film were measured in accordance to the procedures listed below. The results of these measurements are listed in Table II below.

Tensile Test

The film peak load ("CD tensile strength") and elongation at peak load (critical break elongation 90° to the direction of orientation in this case, "critical CD break elongation") were determined in accordance with Method 5102 Federal Test Methods Standard Number 191A. Sample sizes were 7.62cm x 15.24cm (three inches by six inches) with the cross machine direction of the sample running parallel to the 15,24cm (six inch) length of the sample. Three samples were run for each material and the values were averaged. The jaws of the tensile tester were 7.62cm (three inches) wide, the initial gap or gauge length was 7.62cm (three inches) and the crosshead speed was 305mm/min (12 inches per minute).

Water Vapor Transmission Data

The water vapor transmission rate (WVTR) for the sample materials was calculated in accordance with ASTM Standard E96-80. Circular samples measuring 7.62cm (three inches) in diameter were cut from each of the test materials and a control which was a piece of CELGARD® 2500 film from Hoechst Celanese Corporation of Sommerville, New Jersey. CELGARD® 2500 film is a microporous polypropylene film. Three samples were prepared for each material. The test dish was a No. 60-1 Vapometer pan distributed by Thwing-Albert Instrument Company of Philadelphia, Pennsylvania. One hundred milliliters of water were poured into each Vapometer pan and individual samples of the test materials and control material were placed across the open tops of the individual pans. Screw-on flanges were tightened to form a seal along the edges of the pan, leaving the associated test material or control material exposed to the ambient atmosphere over a 6.5 centimeter diameter circle having an exposed area of approximately 33.17 square centimeters. The pans were placed in a forced air oven at 100°F (37°C) for 1 hour to equilibrate. The oven was a constant temperature oven with external air circulating through it to prevent water vapor accumulation inside. A suitable forced air oven is, for example, a Blue M Power-O-Matic 60 oven distributed by Blue M Electric Company of Blue Island, Illinois. Upon completion of the equilibration, the pans were removed from the oven, weighed and immediately returned to the oven. After 24 hours, the pans were removed from the oven and weighed again. The preliminary test water vapor transmission rate values were calculated with Equation (I) below:

(I)      Test WVTR = (grams weight loss over 24 hours) x 315.5 g / m$^2$ / 24hrs

The relative humidity within the oven was not specifically controlled.

Under predetermined set conditions of 100°F (32°C) and ambient relative humidity, the WVTR for the CELGARD® 2500 control has been defined to be 5000 grams per square meter for 24 hours. Accordingly, the control sample was run with each test and the preliminary test values were corrected to set conditions using equation II below:

$$(II) \qquad WVTR = (Test\ WVTR\ /\ control\ WVTR)\ x\ (5000\ g\ /\ m^2\ /\ 24hrs.)$$

TABLE II

| FILM | WVTR (g/m²-24 hours) | CROSS-MACHINE DIRECTION BREAK ELONGATION (%) | CD TENSILE STRENGTH g/3" (7.62 cm) WIDTH |
|---|---|---|---|
| A | 4212 | 261 | 423 |
| B | 4442 | 21 | 435 |
| C | 1742 | 422 | 682 |
| D | 2076 | 345 | 605 |
| E | 2726 | 433 | 617 |
| F | 2403 | 524 | 515 |
| G | 2808 | 390 | 507 |
| H | 3338 | 249 | 744 |

As shown in Table II above, a film of the present invention (films A) has superior cross-machine direction break elongation (a measure of toughness) when compared to film B, which contains the same amount of filler and has similar WVTR value as film A. In addition, although films G and H have increased toughness at the same filler content, their WVTR value values were inferior to that of the film of the present invention (film A). The data relating to films C, D, E, F listed in Table II above show that these films of the present invention may have good controllable WVTR and excellent toughness with lower filler content.

## EXAMPLE 2

Films A, G and H listed in Table I above were each used for preparing laminations. A sheet of absorbent material comprising polypropylene meltblown fibers mixed with pulp fibers, also known as coform, was directed under a spray-head where it was sprayed with a hotmelt adhesive, such as NS-5610 available from National Starch & Chemical Co. of Bridgewater, NJ, at 176,7°C (350°F) temperature at a rate of about 2 grams per square meter. One of the above films was unwound from a roll and lead to a pair of niprolls where the film and the sprayed absorbent were contacted to form a laminate which was then wound into a roll. Subsequently a layer of spunbond of 27,3 g/m² (0.8 ounce per square yard) basis weight was attached to the film side of the laminate by the identical hotmelt laminating process. The WVTR of the three-layer laminates with each of the films was measured and compared to that of the films. The WVTR of the laminate with film A decreased to 4220 g/m²-24 hrs from the film's 4735, a 10.9% drop. The drop with laminate of the G film was 33.4% (to 2143 from 3220), and the drop with laminate of the H film was 28% (to 2278 from 3163). As evident from the above example, the WVTR's of the films containing Catalloy™ (G) or a copolymer of polypropylene (H), already starting from lower values, declined substantially more than the WVTR with film A, the subject of the present invention.

This illustrates the stability of film of the present invention. Films C, D, E and F, not containing the undesirable unstable components were not found to be unstable or otherwise thermally sensitive to declines in breathability.

Therefore, the films of the present invention have high water vapor transmission rate and toughness that impart a wide variety of functionalities including vapor permeability, liquid impermeability, and comfort. Furthermore, such films can be attached to support layers to form laminates.

Of course, it should be understood that a wide range of changes and modifications can be made to the embodiments described above. It is therefore intended that the foregoing description illustrates rather than limits this invention, and that it is the following claims which define this invention.

**Claims**

1.  A stretch-oriented thermoplastic film comprising:

    a filled resin including at least one copolymer of ethylene with at least one $C_4$-$C_8$ -olefin monomer,

    and at least one filler, wherein said at least one filler comprises from 40 to 65 % by weight of said filled resin material of calcium carbonate and wherein said calcium carbonate includes a plurality of particles having a fatty acid coating; and

    wherein said film has a water vapor transmission rate, measured according to the ASTM standard as described in the description, of from 300 to 4,500 g/m$^2$/24 hours; wherein said film has a critical break elongation 90° to the direction of orientation of greater than 150 %.

2.  The film of claim 1, wherein said water vapor transmission rate remained essentially constant after exposing said film to elevated temperatures.

3.  The film of claim 1 wherein:

    said calcium carbonate is present in an amount of 48% by weight of said resin material; and

    said water vapor transmission rate is 1,500 g/m$^2$/24 hours.

4.  The film of claim 1, wherein said film is uniaxially oriented.

5.  The film of claim 4, wherein said filled resin includes a nonelastic material comprising the at least one copolymer and wherein the calcium carbonate has a particle size of from 0.5 to 8 microns.

6.  The film of claim 5, wherein said filled resin includes at least 30 weight percent of said nonelastic material.

7.  The film of claim 5, wherein said nonelastic material includes metallocene-catalyzed ethylene-based copolymers with a density of at least 0.900 g/cc.

8.  A personal care absorbent article comprising a liquid permeable top sheet and a back sheet with an absorbent core disposed therebetween, at least one of said back sheet and said top sheet including the film of claim 1.

9.  The article of claim 8, wherein said article is a diaper.

10. The article of claim 8, wherein said article is a training pant.

11. The article of claim 8, wherein said article is selected from a sanitary napkin or menstrual panty.

12. The article of claim 8, wherein said article is an incontinence device.

13. A process for making a microporous film comprising the steps of:

    providing a resin including a nonelastic material comprising at least one copolymer of ethylene with at least one $C_4$-$C_8$ $\alpha$-olefin monomer;

    adding to said resin material from 40 to 65% by weight of said filled resin material of calcium carbonate wherein said calcium carbonate includes a plurality of particles having a fatty acid coating to form a filled resin;

    extruding said filled resin to form a film;

    stretching said film to form a microporous film; 90°

    wherein said film has a critical break elongation 90° to the direction of orientation of greater than 150%.

14. The process of claim 13, wherein said nonelastic material is provided in an amount of at least 30% by weight of said filled resin.

15. The process of one of claims 13 or 14, wherein said film is uniaxially stretched.

16. The process of claim 13, wherein:

said calcium carbonate is present in an amount of 48% by weight of said filled resin; and

said water vapor transmission rate, measured according to the ASTM standard as described in the description is 1,500 g/m$^2$/24 hours.

17. The process of claim 13, wherein said water vapor transmission rate of said microporous film remained essentially constant after exposing said film to elevated temperatures.

18. A microporous film prepared by the process of claim 13.

19. A breathable laminate comprising:

the thermoplastic film according to any one of claims 1 to 7 and at least one support layer bonded to said film layer.

20. The laminate of claim 19, wherein said support layer is a fibrous nonwoven web.

21. A personal care absorbent article comprising a liquid permeable top sheet and a back sheet with an absorbent core disposed therebetween, at least one of said back sheet and said top sheet including the laminate of claim 19.

22. The article of claim 21, wherein said article is a diaper.

23. The article of claim 21, wherein said article is a training pant .

24. The article of claim 21, wherein said article is selected from a sanitary napkin and a menstrual panty.

25. The article of claim 21, wherein said article is an incontinence device.

26. The article of claim 21, wherein said article is a bandage.

27. A process for forming a breathable laminate comprising:

providing a filled film layer according to any one of claims 1 to 7,

stretching said filled film to produce a microporous film;

bonding at least one support layer to said microporous film to form a laminate.

28. The process of claim 27, wherein said support layer is thermally bonded to said microporous film.

29. The process of claim 27, wherein said support layer is bonded to said microporous film with a hot melt adhesive.

30. The process of one of claims 27 to 29, wherein said filled film is uniaxially stretched.

31. A medical garment comprising the laminate according to any one of claims 19 to 20.

**Patentansprüche**

1. Streckorientierte thermoplastische Folie, umfassend:

ein mit Füllstoff versetztes Harz, umfassend wenigstens ein Copolymer von Ethylen mit wenigstens einem $C_4$-$C_8$-Olefinmonomer,

und wenigstens einen Füllstoff, wobei dieser wenigstens eine Füllstoff 40 bis 65 Gew.% des mit Füllstoff versetzten Harzmaterials Kalziumkarbonat umfasst und wobei das Kalziumkarbonat mehrere Partikel mit einer Fettsäurebeschichtung umfasst; und

wobei die Folie eine Wasserdampf-Durchtrittsrate, gemessen entsprechend ASTM-Standard, wie in der Beschreibung beschrieben, von 300 bis 4.500 g/m$^2$/24 Stunden aufweist; wobei die Folie eine kritische Reißdehnung 90° zur Orientierungsrichtung von mehr als 150 % aufweist.

**2.** Folie gemäß Anspruch 1, wobei die Wasserdampf-Durchtrittsrate im Wesentlichen konstant geblieben ist, nachdem die Folie erhöhten Temperaturen ausgesetzt worden war.

**3.** Folie gemäß Anspruch 1, wobei:

das Kalziumkarbonat in einer Menge von 48 Gewichts% des Harzmaterials vorliegt; und
die Wasserdampf-Durchtrittsrate 1.500 g/m$^2$/24 Stunden beträgt.

**4.** Folie gemäß Anspruch 1, wobei die Folie uniaxial orientiert ist.

**5.** Folie gemäß Anspruch 4, wobei das mit Füllstoff versetzte Harz ein nicht-elastisches Material umfasst, das wenigstens ein Copolymer umfasst, und
wobei das Kalziumkarbonat eine Partikelgröße von 0,5 bis 8 Mikron aufweist.

**6.** Folie gemäß Anspruch 5, wobei das mit Füllstoff versetzte Harz wenigstens 30 Gewichtsprozent des nicht-elastischen Materials umfasst.

**7.** Folie gemäß Anspruch 5, wobei das nicht-elastische Material Metallocen-katalysierte Copolymere auf Ethylenbasis mit einer Dichte von wenigstens 0,900 g/cm$^3$ umfasst.

**8.** Absorbierender Hygieneartikel umfassend eine flüssigkeitsdurchlässige Vorderschicht und eine Unterlagsschicht mit einem absorbierenden Kern, der dazwischen angeordnet ist, wobei wenigstens eine von Unterlagsschicht und Vorderschicht die Folie von Anspruch 1 umfasst.

**9.** Artikel gemäß Anspruch 8, wobei der Artikel eine Windel ist.

**10.** Artikel gemäß Anspruch 8, wobei der Artikel ein Trainingshöschen ist.

**11.** Artikel gemäß Anspruch 8, wobei der Artikel ausgewählt ist aus einer Damenbinde oder einem Menstruationshöschen.

**12.** Artikel gemäß Anspruch 8, wobei der Artikel ein Inkontinenzartikel ist.

**13.** Verfahren zur Herstellung einer mikroporösen Folie, umfassend folgende Schritte:

Bereitstellen eines Harzes umfassend ein nicht-elastisches Material, das wenigstens ein Copolymer von Ethylen mit wenigstens einem $C_4$-$C_8$ α-Olefinmonomer umfasst;

Hinzufügen von 40 bis 65 Gew.% des mit Füllstoff versetzten Harzmaterials Kalziumkarbonat zu dem Harzmaterial, wobei das Kalziumkarbonat mehrere Partikel mit einer Fettsäurebeschichtung aufweist, um ein mit Füllstoff versetztes Harz zu bilden;

Extrudieren des mit Füllstoff versetzten Harzes, um eine Folie zu bilden;

Strecken der Folie, um eine mikroporöse Folie zu bilden;

wobei die Folie eine kritische Reißdehnung 90° zur Orientierungsrichtung von mehr als 150 % aufweist.

**14.** Verfahren gemäß Anspruch 13, wobei das nicht-elastische Material in einer Menge von wenigstens 30 Gew.% des mit Füllstoff versetzten Harzes bereitgestellt wird.

**15.** Verfahren gemäß einem der Ansprüche 13 oder 14, wobei die Folie uniaxial gestreckt wird.

**16.** Verfahren gemäß Anspruch 13, wobei:

das Kalziumkarbonat in einer Menge von 48 Gew.% des mit Füllstoff versetzten Harzes vorliegt; und

die Wasserdampf-Durchtrittsrate, gemessen entsprechend ASTM-Standard, wie in der Beschreibung beschrieben, 1.500 $g/m^2/24$ Stunden beträgt.

**17.** Verfahren gemäß Anspruch 13, wobei die Wasserdampfdurchtrittsrate der mikroporösen Folie im Wesentlichen konstant geblieben ist, nachdem die Folie erhöhten Temperaturen ausgesetzt worden war.

**18.** Mikroporöse Folie, hergestellt durch das Verfahren gemäß Anspruch 13.

**19.** Atmungsaktives Laminat, umfassend:

die thermoplastische Folie gemäß einem der Ansprüche 1 bis 7 und wenigstens eine Trägerlage, die an die Folienlage gebunden ist.

**20.** Laminat gemäß Anspruch 19, wobei die Trägerlage eine Vliesfaserbahn ist.

**21.** Absorbierender Hygieneartikel, umfassend eine flüssigkeitsdurchlässige Vorderschicht und eine Unterlagsschicht mit einem absorbierenden Kern, der dazwischen angeordnet ist, wobei wenigstens eine von Unterlagsschicht und Vorderschicht das Laminat von Anspruch 19 umfasst.

**22.** Artikel gemäß Anspruch 21, wobei der Artikel eine Windel ist.

**23.** Artikel gemäß Anspruch 21, wobei der Artikel ein Trainingshöschen ist.

**24.** Artikel gemäß Anspruch 21, wobei der Artikel ausgewählt ist aus einer Damenbinde und einem Menstruationshöschen.

**25.** Artikel gemäß Anspruch 21, wobei der Artikel ein Inkontinenzartikel ist.

**26.** Artikel gemäß Anspruch 21, wobei der Artikel ein Verband ist.

**27.** Verfahren zur Bildung eines atmungsaktiven Laminates, umfassend:

Bereitstellen einer mit Füllstoff versetzten Folienlage gemäß einem der Ansprüche 1 bis 7,

Strecken der mit Füllstoff versetzten Folie, um eine mikroporöse Folie herzustellen;

Binden von wenigstens einer Trägerlage an die mikroporöse Folie, um ein Laminat zu bilden.

**28.** Verfahren gemäß Anspruch 27, wobei die Trägerlage an die mikroporöse Folie wärmegebunden wird.

**29.** Verfahren gemäß Anspruch 27, wobei die Trägerlage mit einem Heißschmelzkleber an die mikroporöse Folie gebunden wird.

**30.** Verfahren gemäß einem der Ansprüche 27 bis 29, wobei die mit Füllstoff versetzte Folie uniaxial gestreckt wird.

**31.** Medizinisches Bekleidungsstück umfassend das Laminat gemäß einem der Ansprüche 19 bis 20.

**Revendications**

1. Film thermoplastique orienté par étirage comprenant :

   une résine chargée incluant au moins un copolymère d'éthylène avec au moins un monomère α-oléfinique en $C_4$-$C_8$,
   et au moins une charge, ladite au moins une charge comprenant de 40 à 65 % en poids, par rapport audit matériau de résine chargée, de carbonate de calcium et ledit carbonate de calcium comprenant une pluralité de particules ayant un enrobage d'acide gras ; et
   ledit film ayant un débit de transmission de la vapeur d'eau, mesuré selon la norme ASTM indiquée dans la description, compris entre 300 et 4 500 g/m$^2$/24 heures ; et ledit film ayant un allongement à la rupture critique, à 90° par rapport à la direction d'orientation, supérieure à 150 %.

2. Film selon la revendication 1, dans lequel ledit débit de transmission de la vapeur d'eau reste essentiellement constant après exposition dudit film à des températures élevées.

3. Film selon la revendication 1, dans lequel :

   ledit carbonate de calcium est présent en une quantité de 48 % en poids par rapport au matériau de résine ; et
   ledit débit de la transmission de vapeur d'eau est de 1 500 g/m$^2$/24 heures.

4. Film selon la revendication 1, qui est orienté uniaxialement.

5. Film selon la revendication 4, dans lequel ladite résine chargée inclut un matériau non-élastique comprenant le au moins un copolymère et dans lequel le carbonate de calcium a une taille de particule comprise entre 0,5 et 8 microns.

6. Film selon la revendication 5, dans lequel ladite résine chargée inclut au moins 30 % en poids dudit matériau non-élastique.

7. Film selon la revendication 5, dans lequel ledit matériau non-élastique inclut des copolymères à base d'éthylène catalysés par métallocène ayant une masse volumique d'au moins 0,900 g/cm$^3$.

8. Article absorbant d'hygiène intime comprenant une feuille supérieure perméable aux liquides et une feuille dossier, avec un noyau absorbant disposé entre elles, l'une au moins de ladite feuille dossier et de la feuille supérieure incluant le film selon la revendication 1.

9. Article selon la revendication 8 qui est un change pour nourrisson.

10. Article selon la revendication 8 qui est une culotte d'apprentissage de la propreté.

11. Article selon la revendication 8 qui est choisi entre une serviette hygiénique et une culotte périodique.

12. Article selon la revendication 8 qui est un dispositif pour l'incontinence.

13. Procédé de fabrication d'un film microporeux comprenant les étapes suivantes :

    la fourniture d'une résine incluant un matériau non-élastique comprenant au moins un copolymère de l'éthylène avec au moins un monomère α-oléfinique en $C_4$-$C_8$ ;
    l'adjonction audit matériau de résine de 40 à 65 % en poids, par rapport audit matériau de résine chargée, de carbonate de calcium, ledit carbonate de calcium comprenant une pluralité de particules ayant un enrobage d'acide gras, pour former une résine chargée ;
    l'extrusion de ladite résine chargée pour former un film ;
    l'étirage dudit film pour former un film microporeux ;
    ledit film ayant un allongement à la rupture critique, à 90° par rapport à la direction de l'orientation, supérieur à 150 %.

14. Procédé selon la revendication 13, dans lequel ledit matériau non-élastique est fourni en une quantité d'au moins 30 % en poids par rapport à ladite résine chargée.

**15.** Procédé selon l'une des revendications 13 ou 14, dans lequel ledit film est étiré uniaxialement.

**16.** Procédé selon la revendication 13, dans lequel :

ledit carbonate de calcium est présent en une quantité de 48 % en poids par rapport à ladite résine chargée ; et ledit débit de transmission de la vapeur d'eau, mesuré selon la norme ASTM indiquée dans la description, est de 1 500 g/m$^2$/24 heures.

**17.** Procédé selon la revendication 13, dans lequel ledit débit de transmission de la vapeur d'eau dudit film microporeux reste essentiellement constant après exposition dudit film à des températures élevées.

**18.** Film microporeux préparé par le procédé selon la revendication 13.

**19.** Stratifié respirant comprenant :

le film thermoplastique selon l'une quelconque des revendications 1 à 7 et au moins une couche support liée à ladite couche de film.

**20.** Stratifié selon la revendication 19, dans lequel ladite couche support est une nappe non-tissée fibreuse.

**21.** Article absorbant d'hygiène intime comprenant une feuille supérieure perméable aux liquides et une feuille dossier, avec un noyau absorbant disposé entre elles, l'une au moins de ladite feuille dossier et de ladite feuille supérieure incluant le stratifié selon la revendication 19.

**22.** Article selon la revendication 21 qui est un change pour nourrisson.

**23.** Article selon la revendication 21 qui est une culotte d'apprentissage de la propreté.

**24.** Article selon la revendication 21 qui est choisi entre une serviette hygiénique et une culotte périodique.

**25.** Article selon la revendication 21 qui est un dispositif pour l'incontinence.

**26.** Article selon la revendication 21 qui est un pansement.

**27.** Procédé de formation d'un stratifié respirant comprenant :

la fourniture d'une couche de film chargé selon l'une quelconque des revendications 1 à 7 ; l'étirage dudit film chargé pour produire un film microporeux ; la liaison d'au moins une couche support audit film microporeux pour former un stratifié.

**28.** Procédé selon la revendication 27, dans lequel ladite couche support est liée thermiquement audit film microporeux.

**29.** Procédé selon la revendication 27, dans lequel ladite couche support est liée audit film microporeux au moyen d'un adhésif de fusion.

**30.** Procédé selon l'une des revendications 27 à 29, dans lequel ledit film chargé est étiré uniaxialement.

**31.** Vêtement médical comprenant le stratifié selon l'une quelconque des revendications 19 à 20.

FIG.1

FIG. 2

FIG. 3